# FASCICULE DE BREVET EUROPEEN

(11) **EP 0 976 383 B1**
(45) Date de publication et mention de la délivrance du brevet: **22.10.2003**
(21) Numéro de dépôt: 99113705.0
(22) Date de dépôt: 13.07.1999
(51) Int. Cl.: A61K 7/00, A61L 15/00, A61K 9/70

(54) **Procédé de traitement cosmétique de la peau et patch pour la mise en oeuvre du procédé**
Verfahren für die kosmetische Behandlung der Haut und Patch für die Ausführund des Verfahrens
Process for a cosmetic care of the skin and patch for realising said process

(30) Priorité: 31.07.1998 FR 9809880
(43) Date de publication de la demande: 02.02.2000
(73) Titulaire: L'OREAL, 75008 Paris (FR)
(72) Inventeur: Gueret, Jean Louis H., 75016 Paris (FR)
(74) Mandataire: Schmit, Charlotte

(56) Documents cités:
- EP-A- 0 190 814
- EP-A- 0 309 309
- EP-A- 0 412 869
- EP-A- 0 651 984
- EP-A- 0 764 441
- WO-A-98/42303
- US-A- 3 499 446
- US-A- 5 466 456
- US-A- 5 811 107

## Description

La présente invention a pour objet un procédé de traitement cosmétique à l'aide d'un patch, apte à être appliqué sur la peau en deux modes d'application différents, en vue d'obtenir sélectivement, soit une action nettoyante de la peau, soit une action de traitement spécifique. L'invention concerne également un patch pour la mise en oeuvre du procédé.

En particulier, le procédé de traitement de l'invention vise l'application du patch suivant un premier mode d'application destiné à réaliser un nettoyage de la peau, ce même patch étant destiné à permettre un traitement de la peau, obtenu lors de l'application du patch selon un second mode d'application. C'est notamment l'un des objets de l'invention que d'utiliser un seul et unique type de patch pour les deux modes nettoyage et traitement, pour éviter que l'utilisatrice soit obligée d'appliquer deux types de patchs différents, et pour exclure tout risque de confusion entre deux types de patchs.

Ainsi, outre son action nettoyante sur la peau lors de l'application selon le premier mode d'application, le patch visé par la présente invention peut être utilisé pour développer, selon un second mode d'application, d'autres actions, notamment un second traitement suivant lequel la libération sur l'épiderme d'un ou plusieurs composés cosmétiquement actifs est effectuée. Suivant le premier mode d'application visé, de tels patchs sont efficaces, notamment, pour enlever les impuretés présentes sur la surface de la peau, en vue de la préparer pour le second traitement. De telles impuretés peuvent comprendre, des résidus liés à la pollution environnante, des cellules de peau mortes, des bouchons de sébum, des points noirs, des résidus de transpiration, etc. Sur la surface de la peau ainsi nettoyée peut être appliqué ensuite un autre patch du même type, voire le même patch, selon le second mode d'application, destiné à exercer sur la peau une action émolliente, hydratante, adoucissante, cicatrisante, régénérante, anti-rides ou tenseur, protectrice solaire, apaisante, auto-bronzante ou éclaircissante, ou bien à resserrer ou ouvrir les pores de la peau.

Il est connu d'utiliser des patchs permettant, par transdermie, d'administrer des composés actifs, en vue d'un traitement systémique. De tels patchs présentent en général une structure comportant plusieurs couches successives dans l'ordre suivant : une première couche, dite couche support, généralement occlusive ; une seconde couche, dite matrice polymérique, fixée sur la couche support et contenant le (ou les) composé(s) actif(s), cette couche pouvant venir directement au contact de la peau et contenant, éventuellement, pour faciliter la fixation du patch sur la peau, une matière adhésive ; enfin, une pellicule détachable de protection, recouvrant de façon hermétique la couche de polymère, de façon à la protéger de toute contamination extérieure pendant le stockage préalable à l'utilisation du patch.

Par ailleurs, il existe des patchs de soins occlusifs, permettant de faire pénétrer dans l'épiderme, des composés actifs. Ce type de patch est, en général, très peu adhésif, car il comporte des formules aqueuses ou hydrophiles. Ces patchs, destinés au traitement de la peau, sont appliqués, généralement, pendant un temps de pose sur la peau pouvant aller de 15 minutes à 8 heures.

On connaît, également, des patchs de soin occlusifs que l'on applique pendant tout une nuit pour administrer dans l'épiderme un agent actif, ces patchs présentant un pouvoir adhésif sur la peau très élevé. Ces patchs, lors de leur enlèvement, sont agressifs et traumatisants pour la peau, déjà sensibilisée par le traitement préalable, risquant alors de provoquer des gerçures, des rougeurs douloureuses ou des inflammations. Cet effet se produit, notamment, en raison de l'augmentation de l'adhérence du patch sur la peau, en fonction de la durée d'application.

Il existe, d'autre part, des produits du type masque à sec que l'utilisatrice humecte avant application sur la peau. Ce type de masque sèche sur la peau.

Ces masques sont destinés au nettoyage de la peau, notamment des pores, en profondeur.

Il existe aussi des patchs déshydratés, rehumectables à l'eau à base de polymères hydrosolubles, que l'on retire, après séchage, pour desquamer et nettoyer la peau.

On connaît, en outre, des patchs auto-adhésifs comportant une matrice hydrophobe en polyuréthanne, polyacrylate, polyvinyle ou silicone, pourvus de particules absorbeurs d'eau. Les absorbeurs d'eau sont destinés à resolubiliser des actifs hydrosolubles contenus dans la matrice sous forme de suspension. Les patchs de ce genre présentent un pouvoir adhésif relativement limité.

En outre, on connaît des produits secs du type lingette de traitement, non collants, que l'on humidifie et qui ne sèchent pas, pour effectuer un traitement de soin sur le visage.

Aussi, est-ce l'un des objets de l'invention que de réaliser un traitement simple, alterné ou cumulé en deux modes d'application possibles à partir d'un même type de patch. Un autre objet de l'invention est d'autoriser deux résultats de traitement différents, suivant que le patch est appliqué selon un premier mode d'application à sec, ou selon un second mode d'application à l'état mouillé.

C'est un autre objet de l'invention que de fournir un patch qui incorpore différents composés cosmétiquement actifs, qui, outre leur action pour débarrasser la peau de ses impuretés, sous quelle forme que ce soit, ont une seconde action, notamment hydratante, adoucissante, émolliente, cicatrisante, régénérante, apaisante, auto-bronzante, éclaircissante, anti-rides, protectrice contre les rayons solaires, ou une action réductrice de graisse.

C'est en particulier l'un des objets de l'invention que de préparer la peau lors d'une étape de nettoyage, apte à faciliter l'administration d'un traitement de la peau, afin de rendre ce dernier plus efficace.

Dans ce but, un autre objet encore de l'invention consiste à proposer un traitement simplifié, ne mettant en oeuvre qu'un seul type de patch, applicable en deux modes d'application différents.

Aussi, la présente invention a pour objet un procédé de traitement cosmétique de la peau au moyen d'un patch constitué d'une matrice polymérique déposée sur un support, ladite matrice étant auto-adhésive sur peau sèche, et contenant au moins un composé cosmétiquement actif, et au moins un composé hydroabsorbant, ledit procédé consistant à sélectivement, appliquer le patch :
a) en mode nettoyage sur peau sèche, le décollement du patch après application permettant, sous l'action mécanique résultant de l'adhérence du patch, de débarrasser la peau des impuretés qu'elle contient, ou
b) en mode traitement à l'état mouillé, le (ou les) composé(s) hydroabsorbants permettant d'une part, en asséchant partiellement la peau, de conserver une adhérence de la matrice sur la peau, laquelle adhérence est réduite par rapport à adhérence de la matrice sur peau sèche, et d'autre part, en absorbant de l'eau, de resolubiliser tout ou partie des composés cosmétiquement actifs contenus dans la matrice pour les mettre en contact avec la peau pendant toute la durée d'application.

Par l'expression "à l'état mouillé", on entend un mode de traitement suivant lequel, soit la peau, soit la surface du patch destinée à adhérer à la peau, est préalablement mouillée, notamment à l'eau. Le mouillage de la peau constitue cependant le mode d'application préféré.

Ainsi sur peau sèche, le patch colle parfaitement à la peau, grâce à la présence de la matrice auto-adhésive, en contact sur sensiblement toute la surface du patch avec la peau. Lors de l'enlèvement du patch, les impuretés se trouvant à la surface de la peau, et dont la libération peut être favorisée par la présence de certains actifs, sont arrachés sous l'action mécanique de l'adhésif. Eventuellement, des actifs peuvent être libérés sur la peau lors de l'arrachage du patch. A l'état mouillé, les hydroabsorbants, séparés de la surface libre du patch, par une très fine couche de matrice adhésive, absorbent de l'eau, en formant notamment un gel, ou un réseau de fibres gorgées d'eau, dans lequel les actifs vont se solubiliser, et pouvoir être mis en contact avec la peau. En outre, en absorbant une partie de l'eau, il subsiste un certain nombre de points d'ancrages entre la matrice adhésive et la peau, de manière à autoriser une adhérence suffisante du patch sur la peau, laquelle adhérence est sensiblement réduite par rapport à l'adhérence sur peau sèche.

Un autre objet de l'invention se rapporte à un patch, utilisable pour la mise en oeuvre du procédé de traitement défini ci-dessus.

Selon un mode préféré de l'invention, ce procédé consiste en une application en mode nettoyage, suivie d'une application en mode traitement. Il est possible d'effectuer ces deux applications, immédiatement l'une après l'autre. Cependant, avantageusement, on permet à la peau de se reposer, par exemple pendant une durée comprise entre 30 minutes et 12 heures.

Selon une autre possibilité, on effectue sur la peau d'abord l'application du patch en mode traitement, suivie d'une application en mode nettoyage. Ainsi, par un traitement émollient, on ouvre les pores pour faciliter, ensuite, l'opération de nettoyage en profondeur.

Généralement, l'application du patch en mode nettoyage est réalisée en alternance avec l'application en mode traitement. Il peut être avantageux, cependant, notamment lorsque la peau est grasse, de réaliser une pluralité d'applications en mode nettoyage, en alternance avec une pluralité d'applications en mode traitement.

De préférence, le patch visé par la présente invention est utilisé pendant une durée d'application, en mode nettoyage, allant d'environ 5 secondes à environ 5 minutes.

En mode traitement, l'application peut être effectuée pendant une durée allant d'environ 5 minutes à environ 30 minutes, voire 60 minutes si besoin est.

Les composés cosmétiquement actifs qui peuvent être incorporés dans la matrice du patch, conformément à l'invention, sont choisis, avantageusement, parmi les agents émollients, hydratants, adoucissants, kératolytiques, desquamants, cicatrisants, régénérants, anti-rides, tenseurs, protecteurs solaires, apaisants, auto-bronzants, dépigmentants, éclaircissants ou leurs mélanges. De plus, on peut incorporer dans la matrice polymérique des agents antioxydants, anti-radicaux libres, liporégulateurs, anti-acnéiques, anti-vieillissement, anti-inflammatoires, rafraîchissants, protecteurs vasculaires, antibactériens, antifongiques ou nourrissants.

Selon un mode de mise en oeuvre avantageux de l'invention, le(s) composé(s) cosmétiquement actif(s) est(sont) dispersé(s), de manière homogène, dans la matrice polymérique sous forme particulaire. Ceci est intéressant, notamment, lors de l'application du patch en mode traitement sur peau mouillée, car l'actif n'est solubilisé dans la matrice substantiellement, qu'au moment où sa pénétration doit intervenir.

Le composé cosmétiquement actif, dont on se sert notamment en mode nettoyage sur peau sèche, peut être, avantageusement, un composé ayant une action permettant de détacher les impuretés de la peau (par exemple, les agents kératolytiques, qui décollent les cellules de peau morte), le prélèvement sur le patch se faisant ensuite par action mécanique de l'adhésif. Il peut s'agir également d'un ou plusieurs composés contenus dans la matrice, et aptes à absorber directement les sécrétions grasses ou aqueuses de la peau telles que le sébum ou la transpiration. A cet effet, on peut utiliser des matières telles que la poudre de Kaolin, la terre de Sienne, des zéolites, des particules de polyamide (notamment celles vendues sous la dénomination "ORGASOL®" par la société ATOCHEM), ces dernières ayant en outre une action adoucissante au contact de la peau. On peut citer, également, la poudre de micro-billes thermoplastiques expansées, vendus sous la dénomination commerciale "EXPANCEL®" par la société KEMANORD PLAST, ou les poudres d'amidon modifié, commercialisées par la société NATIONAL STARCH sous la dénomination commerciale "Dry Flo®". On peut utiliser aussi d'un mélange de telles matières.

Les composés actifs à effet nettoyant pouvant être incorporés dans la matrice, incluent notamment les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique, et de manière générale, les acides de fruits et les bêta-hydroxyacides, comme l'acide salicylique et ses dérivés, notamment alcoylés, comme l'acide n-octanoyl-5-salicylique est ses esters. On peut également utiliser des antibactériens, tels que le phosphate de clindamicyne, l'érythromycine ou les antibiotiques de la classe des tétracyclines. De tels actifs sont indiqués lorsque la peau a une tendance acnéique, et peuvent être utilisés en combinaison notamment avec des agents émollients, des agents adoucissants, notamment du miel ou des cires, et/ou des agents cicatrisants, notamment certains sels minéraux, comme l'oxyde de zinc. On peut également associer des agents astringents, tels que les tanins ou les chlorohydrates d'aluminium ou de zirconium.

On peut utiliser, notamment, un ou plusieurs actifs choisis notamment parmi l'acide ascorbique et ses sels et esters biologiquement compatibles, les enzymes, les composants à effet tenseur, tels que les poudres de protéines, de soja ou de blé, les polyacides hydroxylés, les sucroses et leurs dérivés, l'urée, les aminoacides, les oligopeptides, les extraits hydrosolubles végétaux et de levures, les hydrolysats de protéines telles que la collagène et l'élastine, l'acide hyaluronique, les mucopolysaccharides, les vitamines B₂, B₆, H, PP, le panthénol, l'acide folique, l'acide acétyl salicylique, l'allantoïne, l'acide kojique, l'hydroquinone, etc..

La matrice peut également comporter au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches en acides gras essentiels ; acide asiatique, acide madécassique, asiaticoside, extrait total de centella asiatica, acide β-glycyrrhétinique, α-bisabolol, céramides comme le 2-oleoylamino-1,3 octadécane ; phytanetriol, sphingomyéline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

A titre d'exemple, l'agent émollient est choisi parmi : les silicones volatils ou non volatils, les copolymères polydiorganosiloxane-polyoxyalkylène ; les huiles naturelles ou synthétiques comme les huiles minérales, végétales et animales ; les graisses et les cires ; les alcools et acides gras et leurs esters ; les esters et éthers de (poly)alkylène glycols ; les hydrocarbures comme l'isohexadécane, le petrolatum, le squalane ; l'alcool lanolique et ses dérivés ; les triglycérides animaux et végétaux ; l'alcool stéarylique.

L'agent hydratant est choisi, avantageusement, parmi : le glycérol, le sorbitol et ses dérivés, le propylène glycol, le dipropylène glycol, le butylène glycol, le D-, ou DL-panthénol et leurs dérivés.

De préférence, on choisit comme agent adoucissant l'allantoïne.

L'agent desquamant peut être choisi parmi : les dérivés alcoylés de l'acide salicylique, comme l'acide n-octanoyl-5-salicylique, les acides alpha- et bêta-hydroxy-carboxyliques ou bêta-cétocarboxyliques, leurs sels, amides ou esters.

Les agents anti-rides sont choisis, de préférence, parmi les acides alpha- et bêta-hydroxy-carboxyliques ou bêta-cétocarboxyliques, leurs sels, amides ou esters ; l'acide salicylique, l'acide ascorbique, l'acide azélaique, le rétinol et leurs dérivés.

Lorsque des agents protecteurs solaires sont présents, ceux-ci sont choisis parmi les filtres UV-A et/ou les filtres UV-B utilisés habituellement dans les compositions anti-solaires.

Comme agent apaisant on choisit par exemple l'α-bisabolol.

Lorsqu'on utilise un agent auto-bronzant, ce dernier est choisi parmi la dihydroxy acétone et les dérivés d'hydroxy naphthoquinone.

Avantageusement, l'agent éclaircissant ou dépigmentant est choisi parmi l'acide citrique, l'hydroquinone, l'acide kojique, l'acide ascorbique.

Généralement, la matrice polymérique est constituée d'une matrice à base d'adhésif siliconné, ou de préférence, à base d'adhésif polyacrylique ou polyvinylique. L'adhérence du patch selon l'invention sur peau sèche (lorsqu'on est en mode nettoyage) est typiquement comprise entre environ 300 g/cm² et environ 800 g/cm² (force exercée perpendiculairement au plan de la surface adhésive, nécessaire à son décollement de la peau), son adhérence sur peau mouillée étant de préférence comprise entre environ 20 g/cm² et environ 300 g/cm². La surface de la matrice destinée à venir en contact avec la peau peut être lisse ou présenter des aspérités ou reliefs. L'épaisseur de la matrice peut être variable, notamment en fonction de la quantité utilisée en composés actif(s) et hydroabsorbant(s). Typiquement, cette épaisseur peut varier entre 5 µm et 500 µm.

Selon une caractéristique intéressante de l'invention, après évaporation des solvants, au moins certaines des particules d'actifs ou d'agents hydroabsorbants ou les agglomérats formés par de telles particules, émergent par rapport à la surface moyenne de la matrice adhésive. Dans la pratique, ces particules n'émergent généralement pas directement, mais en réalité sont enrobées d'une très fine couche de ladite matrice adhésive. A titre indicatif, les particules émergent d'une hauteur pouvant aller de quelques microns à quelques centaines de microns, et de préférence, de 10 microns à 100 microns, et de préférence encore, de 10 µm à 70 µm. De préférence, l'épaisseur moyenne de la matrice (après évaporation des solvants) est inférieure à la plus grande dimension des charges ou particules d'actifs ou composés hydroabsorbants, présentes dans la matrice. Le cas échéant, si lesdites charges ou particules forment des agglomérats, l'épaisseur moyenne de la matrice est inférieure à la plus grande dimension desdits agglomérats. Cette disposition permet, en mode traitement à l'état mouillé, d'obtenir la formation d'une couche interface formée par les hydroabsorbants gorgés d'eau, notamment sous forme d'un gel, entre la matrice adhésive et la peau, et dans laquelle peuvent se solubiliser les actifs, lesquels vont ainsi pouvoir être mis en contact avec la peau. L'épaisseur de la couche interface formée au dessus de la matrice adhésive peut aller avantageusement de 50µm à 100 µm. Ainsi, seule une faible portion de la matrice adhésive est en contact avec la peau, assurant ainsi une adhérence réduite sur la peau, suffisante toutefois, éventuellement en combinaison avec l'adhérence intrinsèque du gel, pour assurer le maintien du patch sur la peau.

Avantageusement, le(s) agent(s) hydro-absorbant(s) incorporés dans la matrice polymérique est(sont) constitué(s) de particules dispersées dans ladite matrice. En effet, au contact de l'eau présente sur la peau, notamment après mouillage, les particules de l'agent hydro-absorbant captent de l'eau, favorisant ainsi la solubilisation d'un composé actif solide, hydrosoluble. En quelque sorte, par cette solubilisation "in situ" de l'actif hydrosoluble, sa biodisponibilité est quasi instantanée, et toute interaction éventuelle avec les autres composés présents dans la matrice polymérique est minimisée. L'eau présente sur la peau (ou sur le patch avant application) joue le rôle de solubilisant de l'actif hydrosoluble.

Parmi les agents hydro-absorbants présents dans la matrice polymérique à l'état dispersé, on peut citer, de préférence, les polyacrylates réticulés superabsorbants à fort taux de gonflement dans l'eau, tels que ceux commercialisés par la Société NORSOLOR sous la dénomination "AQUAKEEP®" ; l'alcool polyvinylique ; les polymères carboxyvinyliques tels que ceux commercialisés par la Société GOODRICH sous les dénominations de "CARBOPOL®" ; les dérivés semi-synthétiques de la cellulose tels que la carboxyméthylcellulose les amidons, les biogommes telles que les gommes de xanthane, de guar, les gommes arabique et adragante, les biosaccharides, les scléroglucanes ; la caséine ; les phycocolloïdes, comme les alginates, les carragénates, l'agar-agar; la gélatine et les fibres de coton.

On préfère, tout particulièrement, utiliser les polyacrylates réticulés superabsorbants dont la présence à l'état dispersé dans une matrice polymérique hydrophobe permet d'accumuler un taux important d'eau, et favorise, après hydratation, une meilleure disponibilité des particules des autres composés actifs présents, le cas échéant, dans la matrice.

L'agent hydro-absorbant tel que défini ci-dessus est présent, de préférence, dans une proportion allant d'environ 0,2 % à environ 20 % en poids, et plus particulièrement allant de 0,5 % à 10 % par rapport au poids total de la matrice polymérique.

On peut inclure, dans la matrice polymérique des huiles essentielles. On citera, à titre d'exemple l'huile de lavande, de menthe, de poivre, de muscade, de clou de girofle, de cèdre, de germes de blé, de calophyllum, de carthame, de coriandre, de pepins de fruits (raisin, cassis, orange, kiwi). L'huile de menthe contient un taux élevé en menthol, qui, inclus dans la matrice sous forme microcristalline, procure une sensation de fraîcheur sur la peau.

Le support selon la présente invention peut être constitué de tout matériau approprié imperméable aux composés actifs contenus dans la matrice polymérique adjacente. Le support a non seulement pour fonction de supporter la couche de polymère mais également de servir de revêtement protecteur de celle-ci. Il peut être de même dimension que la couche de polymère ou de dimension plus grande de telle sorte qu'il s'étende au-delà la périphérie de la matrice polymérique.

Le support peut être un support occlusif. A titre d'exemple, le support est constitué d'un matériau thermoplastique, choisi parmi les polyéthylènes haute et basse densité, les polypropylènes, les polychlorures de vinyle, les copolymères d'éthylène et d'acétate de vinyle, les polyesters et les polyuréthannes, le polyéthylène téréphthalate, ou d'un complexe de tels matériaux. Ces matériaux peuvent également être présents sous forme stratifiée avec au moins une feuille de métal telle qu'une feuille d'aluminium.

Le support peut être de toute épaisseur appropriée pour assurer les fonctions de support et de protection. De préférence, l'épaisseur du support est comprise entre environ 20 µm et environ 1,5 mm. Avantageusement, le support est suffisamment flexible de manière à pouvoir épouser parfaitement le profil de la peau, et à ne pas provoquer chez l'utilisateur, une sensation d'inconfort.

En variante, le support peut être non occlusif. Dans cette dernière hypothèse, on utilise avantageusement un support constitué d'un papier, d'une trame d'un matériau thermoplastique poreux ou perforé, d'un tissé, d'un non tissé, d'un non tissé perforé. Avantageusement, lorsqu'on utilise une trame, celle-ci peut être noyée dans la matrice polymérique.

Avantageusement encore, le support du patch comprend au moins une excroissance de faible dimension, sous forme d'un onglet, apte à favoriser le décollement du patch.

Les patchs selon la présente invention peuvent, en outre, être protégés par la présence d'une pellicule détachable ou pelable de protection adjacente à la matrice polymérique et/ou par un conditionnement dans un emballage approprié, notamment imperméable à l'eau et à la vapeur d'eau. La pellicule peut avoir une surface lisse ou gaufrée. Dans le cas d'une pellicule à surface gaufrée, on diminue son adhérence sur la matrice, favorisant ainsi le décollement de la pellicule.

Lorsque la matrice polymérique est protégée par une pellicule détachable de protection, celle-ci est enlevée au moment de l'utilisation. Elle peut être constituée en tout matériau imperméable au composé actif ainsi qu'à tout autre composant présent dans la matrice polymérique. Parmi les matériaux pouvant être utilisés, on peut citer de préférence, une feuille de papier siliconée ou une feuille de matériau thermoplastique traitée pour la rendre anti-adhérente, par exemple à l'aide de silicone ou d'un vernis. De préférence, cette pellicule détachable de protection est constituée de polyéthylène. Avantageusement, la feuille de protection est constituée de deux parties se superposant sur une portion médiane du patch, de manière à en faciliter la pose sans que les doigts viennent en contact avec la matrice contenant le ou les actifs. Selon une alternative, la feuille de protection s'étend sur une surface supérieure à la surface du patch, et déborde au delà des limites de ce dernier, de manière à en favoriser le décollement.

Selon une forme de réalisation particulièrement intéressante, la matrice polymérique peut être colorée de manière à pouvoir, en mode nettoyage, de visu, quantifier et/ou qualifier les impuretés prélevées, par la surface adhésive du patch.

A cet effet, dans la matrice polymérique du patch peuvent être incorporés des pigments colorés. Dans ce cas, il est avantageux que la couleur de la matrice polymérique soit foncée, de manière à réaliser un contraste suffisant pour mettre en évidence les impuretés extraites de la peau. Ainsi, une fois décollé de la peau, le patch porte sur sa surface adhésive colorée, toutes les impuretés qui ont été extraites lors du traitement. Ainsi, lorsque le patch est appliqué sur peau sèche, l'adhésif assure une fonction de "peeling" de la peau.

La quantité d'impuretés prélevées est représentative de la fréquence souhaitable d'application dudit patch. Ainsi, la présence d'une quantité importante de tels résidus indique à l'utilisatrice qu'elle doit appliquer le patch sur la base d'une fréquence relativement élevée (par exemple, tous les jours). Une quantité plus faible de telles impuretés indique que la fréquence d'utilisation doit être plus faible (par exemple, une fois par semaine). En d'autres termes, en mode nettoyage, un patch coloré permet de quantifier l'efficacité du traitement qu'il apporte. En outre, le type d'impuretés prélevées permet à l'utilisatrice de choisir au mieux la durée d'application, à la suite, en mode traitement.

Les pigments colorés peuvent être constitués notamment de pigments du type de ceux utilisés dans le domaine de l'alimentaire ou de la cosmétique, en particulier pour les rouges à lèvres ou les vernis à ongles. A titre d'exemples, on peut citer, seuls ou en combinaison, des pigments synthétiques, ou des pigments minéraux, notamment des pigments d'oxyde de zirconium ou de cérium, ainsi que les oxydes de fer ou de chrome, et le bleu ferrique. On peut utiliser des pigments organiques, notamment le noir de carbone, les laques de baryum, zirconium (D&C Red No. 9), strontium (D&C Red No. 6), calcium (D&C Red No. 7), aluminium ; les pigments azoïques et anthraquinoniques (D&C violet No. 2).

De façon connue, les patchs selon la présente invention peuvent être découpés selon un contour approprié, correspondant à la zone de surface de peau à traiter, par exemple sous forme de masque pour l'application sur le visage, notamment pour l'application sur le nez, les lèvres, les joues, la zone entre le nez et la lèvre supérieure, ou pour traiter les pattes d'oie du coin externe de l'oeil, les poches en-dessous de l'oeil, ou les rides du front. Bien entendu, les patchs selon la présente invention peuvent être découpés sous toute autre forme nécessaire pour une application sur une zone déterminée du corps. En général, la taille d'un patch conforme à l'invention est comprise entre 0,25 cm² et 500 cm², et de préférence, entre 1 cm² et 30 cm².

Les patchs ainsi constitués et découpés peuvent être utilisés, après élimination de la couche détachable de protection, sur une surface de peau à traiter, en les appliquant directement sur une zone de peau sèche pour exercer une action nettoyante. Lors de cette application des actifs favorisant le décollage des impuretés de la peau et l'enlèvement des cellules mortes, pénètrent dans l'épiderme et les couches plus profondes de la peau. Grâce à la forte adhérence du patch à sec, lors de l'enlèvement du patch, ces impuretés et cellules mortes restent collées sur la surface apparente du patch. L'enlèvement du patch peut être accompagné d'une légère irritation de la peau, se manifestant par l'apparition d'une faible rougeur, provoquant une augmentation de l'irrigation sanguine du derme. Ceci a pour conséquence une meilleure diffusion des actifs préalablement migrés dans l'épiderme, vers les couches plus profondes du derme, voire, à long terme, une stimulation de l'angiogenèse cutanée.

Suite au décollage du patch, après une période de repos appropriée, l'utilisatrice applique un second patch du même type sur la même zone de peau, préalablement mouillée à l'eau, pour effectuer son application en mode traitement. Généralement, la durée d'application en mode traitement est plus longue que la durée d'application en mode nettoyage. Après une durée d'application appropriée, les actifs traitants sont solubilisés dans la couche interface formée au dessus de la matrice adhésive et sont mis au contact de la peau. L'adhérence du patch, pendant cette phase de traitement est assurée par la présence des composés hydro-absorbants qui remplissent une fonction double. En effet, d'une part, ils retiennent une quantité nécessaire en eau pour la mise en solution des composés de traitement actifs, et d'autre part, ils créent des points d'ancrage anhydres à la surface de la matrice permettant à celle-ci, d'exercer son pouvoir adhésif vis-à-vis de la surface de la peau. Après une durée de pose appropriée, le patch peut être enlevé. Ainsi, on peut engager un autre cycle d'application en temps voulu. Le traitement peut être modulé de sorte qu'il convienne au mieux au besoin de la peau à traiter, quant à la fréquence et la durée d'application.

Ainsi, on réalise un patch pouvant être appliqué selon deux modes différents : un mode nettoyage et un mode traitement. Les actifs agissant le cas échéant en mode nettoyage peuvent ne pas être les mêmes que ceux agissant en mode traitement. Les séquences d'application selon l'un ou l'autre de ces deux modes peuvent être choisis à volonté en fonction des besoins de la peau.

### EXEMPLE D'APPLICATION

On réalise un patch comportant:
a) une feuille de polyéthylène constituant le support du patch et présentant une épaisseur de 200 µm ;
b) une matrice polymérique réalisée sous forme d'une couche de 0,2 mm d'épaisseur moyenne comportant :

| | |
|---|---|
| acide ascorbique | 1,5 % |
| menthol | 0,5 % |
| essence de lavande | 0,1 % |
| acide lactique | 5 % |
| Poudre de polyamide (ORGASOL®) | 5 % |
| acide citrique | 1,5 % |
| allantoïne | 2 % |
| polyacrylate hydroabsorbant (AQUAKEEP®) | 5 % |
| polymère acrylique autoadhésif qsp. | 100 % |

Cette matrice présente un pouvoir adhésif sur peau sèche d'environ 500 g/cm² ;
c) une feuille de papier siliconnée constituant la pellicule de protection détachable.

L'observation de la surface de la matrice à l'état sec montre la présence de nombreux reliefs (formés par des particules d'actifs ou d'hydroabsorbants, ou d'agglomérats de telles particules) disséminés de façon plus ou moins régulière sur la surface du patch, et faisant saillie par rapport à la surface moyenne de la couche sur une hauteur d'environ 50 µm.

Un patch ainsi constitué, pour effectuer une application sur le front, est découpé par exemple en forme de rectangle de 3 cm x 8 cm.

Lors de l'utilisation, en mode nettoyage, le patch, après enlèvement de la pellicule de protection détachable, est appliqué directement sur le front, pendant une période de 5 minutes environ. Pendant cette période, en raison de la présence de l'acide lactique, le décollement des cellules de peau morte en surface est favorisé. Lorsqu'après cette période, le patch est retiré, les cellules mortes restent accrochées à la matrice adhésive.

Après un repos de 30 minutes, on mouille le front avec de l'eau. On applique alors un second patch du même type sur le front mouillé, et on laisse poser pendant 30 minutes. Au bout de cette période, après décollage du second patch, on peut observer un éclaircissement de la zone traitée, lié à la présence d'acide ascorbique et d'acide citrique. Par ailleurs, grâce à l'action de l'allantoïne, la peau de la zone traitée a gagné en souplesse, et présente plus de douceur au toucher. On constate, en outre, un estompage de ridules du front.

## Revendications

1. Procédé de traitement cosmétique de la peau au moyen d'un patch constitué d'une matrice polymérique déposée sur un support, ladite matrice étant auto-adhésive sur peau sèche, et contenant au moins un composé cosmétiquement actif, et au moins un composé hydroabsorbant, ledit procédé consistant à sélectivement, appliquer le patch :
a) en mode nettoyage sur peau sèche, le décollement du patch après application permettant, sous l'action mécanique résultant de l'adhérence du patch, de débarrasser la peau des impuretés qu'elle contient, ou
b) en mode traitement à l'état mouillée, le (ou les) composé(s) hydroabsorbants permettant d'une part, en asséchant partiellement la peau, de conserver une adhérence de la matrice sur la peau, laquelle adhérence est réduite par rapport à adhérence de la matrice sur peau sèche, et d'autre part, en absorbant de l'eau, de resolubiliser tout ou partie des composés cosmétiquement actifs contenus dans la matrice pour les mettre en contact avec la peau pendant toute la durée d'application.

2. Procédé selon la revendication 1, **caractérisé en ce que** le support est occlusif.

3. Procédé selon la revendication 1 ou 2, **caractérisé en ce que** le support est réalisé en un matériau thermoplastique, choisi parmi les polyéthylènes haute et basse densité, les polypropylènes, les polychlorures de vinyle, les copolymères d'éthylène et d'acétate de vinyle, les polyuréthannes et les polyesters, ou en un complexe de tels matériaux ou le polyéthylène téréphthalate couvert d'une feuille en aluminium.

4. Procédé selon la revendication 1, **caractérise en ce que** le support est non occlusif.

5. Procédé selon la revendication 4, **caractérisé en ce que** le support est réalisé en papier, en un matériau thermoplastique poreux ou perforé, en un tissé, en un non-tissé, en un non-tissé perforé.

6. Procédé de traitement selon l'une quelconque des revendications précédentes, **caractérisé en ce qu'**il consiste en une application en mode nettoyage, suivie d'une application en mode traitement.

7. Procédé selon l'une quelconque des revendications 1 à 5, **caractérisé en ce qu'**il consiste en une application en mode traitement, suivie d'une application en mode nettoyage.

8. Procédé de traitement selon l'une quelconque des revendications précédentes, **caractérisé en ce que** le composé cosmétiquement actif est choisi parmi les agents émollients, hydratants, adoucissants, kératolytiques, desquamants, cicatrisants, régénérants, anti-rides, tenseurs, protecteurs solaires, apaisants, auto-bronzants, éclaircissants ou leurs mélanges.

9. Procédé de traitement selon l'une quelconque des revendications précédentes, **caractérisé en ce que** le composé cosmétiquement actif est dispersé dans la matrice polymérique sous forme particulaire.

10. Procédé de traitement selon l'une quelconque des revendications précédentes, **caractérisé en ce que** l'application en mode nettoyage est réalisée en alternance avec l'application en mode traitement.

11. Procédé de traitement selon la revendication précédente, **caractérisé en ce que** l'on réalise une pluralité d'applications en mode nettoyage en alternance avec une pluralité d'applications en mode traitement.

12. Procédé de traitement selon l'une quelconque des revendications précédentes, **caractérisé en ce que** l'application en mode nettoyage est effectuée pendant une durée allant d'environ 5 secondes à environ 5 minutes.

13. Procédé de traitement selon l'une quelconque des revendications précédentes, **caractérisé en ce que** l'application en mode traitement est effectuée pendant une durée allant d'environ 5 minutes à environ 30 minutes.

14. Procédé de traitement selon l'une quelconque des revendications précédentes, **caractérisé en ce que** le patch a un pouvoir adhésif sur peau seche compris entre environ 300 et environ 800 g/cm².

15. Procédé de traitement selon l'une quelconque des revendications précédentes, **caractérisé en ce que** le patch a un pouvoir adhésif à l'état mouillé compris entre environ 20 et environ 300 g/cm².

16. Procédé de traitement selon l'une quelconque des revendications précédentes, **caractérisé en ce que** le composé cosmétiquement actif est choisi parmi les actifs hydrosolubles tels que l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits hydrosolubles végétaux et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

17. Procédé de traitement selon l'une quelconque des revendications précédentes, **caractérisé en ce que** le composé cosmétiquement actif est choisi parmi les composés liposolubles tels que D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

18. Procédé de traitement selon la revendication 8, **caractérisé en ce que** l'agent kératolytique est choisi parmi : les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique.

19. Procédé de traitement selon la revendication 8, **caractérisé en ce que** l'agent émollient est choisi parmi : les silicones volatils ou non volatils, des copolymères polydiorganosiloxane-polyoxyalkylène ; les huiles naturelles ou synthétiques comme les huiles minérales, végétales et animales ; les graisses et les cires ; les alcools et acides gras, et leurs esters ; les esters et éthers de (poly)alkylène glycols ; les hydrocarbures comme l'isohexadécane, le petrolatum, le squalane ; l'alcool lanolique et ses dérivés ; les triglycérides animales et végétales ; l'alcool stéarylique.

20. Procédé de traitement selon la revendication 8, **caractérisé en ce que** l'agent hydratant est choisi parmi : le glycérol, le sorbitol et ses dérivés, le propylène glycol, le dipropylène glycol, le butylène glycol : D-, ou DL-panthénol et leurs dérivés.

21. Procédé de traitement selon la revendication 8, **caractérisé en ce que** l'agent adoucissant est choisi parmi : l'allantoïne.

22. Procédé de traitement selon la revendication 8, **caractérisé en ce que** l'agent desquamant est choisi parmi : les dérivés alcoylés de l'acide salicylique l'acide n-octanoyl-5-salicylique, les acides alpha- et bêta-hydroxy-carboxyliques ou bêta-cétocarboxyliques, leurs sels, amides ou esters.

23. Procédé de traitement selon la revendication 8, **caractérisé en ce que** les agents anti-rides et tenseur sont choisis parmi : les acides alpha- et bêta-hydroxy-carboxyliques ou bêta-cétocarboxyliques, leurs sels, amides ou esters ; l'acide salicylique, l'acide ascorbique, l'acide azélaïque, le rétinol et leurs dérivés.

24. Procédé de traitement selon la revendication 8, **caractérisé en ce que** l'agent protecteur solaire est choisi parmi les filtres UV-A et/ou les filtres UV-B.

25. Procédé de traitement selon la revendication 8, **caractérisé en ce que** l'agent apaisant est choisi parmi : α-bisabolol.

26. Procédé de traitement selon la revendication 8, **caractérisé en ce que** l'agent auto-bronzant est choisi parmi : la dihydroxy acétone et les dérivés d'hydroxy naphthoquinone.

27. Procédé de traitement selon la revendication 8, **caractérisé en ce que** l'agent éclaircissant est choisi parmi : l'acide citrique, l'hydroquinone, l'acide kojique, l'acide ascorbique.

28. Procédé de traitement selon l'une quelconque des revendications précédentes, **caractérisé en ce que** la matrice polymérique du patch contient un polymère acrylique ou vinylique.

29. Procédé de traitement selon l'une quelconque des revendications précédentes, **caractérisé en ce que** le composé hydroabsorbant est choisi parmi les polyacrylates réticulés superabsorbants ; l'alcool polyvinylique ; les polymères carboxyvinyliques ; les dérivés semi-synthétiques de la cellulose ; les amidons ; les biogommes telles que les gommes de xanthane, de guar, les gommes arabique et adragante, les biosaccharides, les scléroglucanes ; la caséine ; les phytocolloïdes, comme les alginates ; la gélatine et les fibres de coton.

30. Procédé de traitement selon l'une quelconque des revendications précédentes, **caractérisé en ce que** le patch comprend au moins une excroissance, apte à former une zone de préhension pour son décollement.

31. Procédé de traitement selon l'une quelconque des revendications précédentes, **caractérisé en ce que** la matrice polymérique est colorée de manière à pouvoir, de visu, quantifier et/ou qualifier les impuretés prélevées par ladite surface adhésive, en mode nettoyage.

32. Procédé de traitement selon la revendication 31, **caractérisé en ce que** la matrice polymérique du patch est colorée par incorporation de pigments colorés.

33. Procédé de traitement selon la revendication 31 ou 32, **caractérisé en ce que** la couleur de la matrice polymérique est foncée, de manière à réaliser un contraste suffisant pour mettre en évidence les impuretés extraites de la peau.

34. Procédé de traitement selon l'une quelconque des revendications précédentes, **caractérisé en ce que** la matrice polymérique comporte une trame, réalisée en un matériau thermoplastique poreux ou perforé, en un tissé, en un non-tissé, en un non-tissé perforé.

35. Procédé de traitement selon l'une quelconque des revendications précédentes, **caractérisé en ce que** le patch comprend une feuille de protection, pelable avant application du patch, et imperméable au(x) composé(s) entrant dans la composition de la couche polymérique.

36. Procédé de traitement selon la revendication 35, **caractérisé en ce que** la feuille de protection du patch est constituée de deux parties se superposant sur une portion médiane du patch.

37. Procédé de traitement selon l'une quelconque des revendications précédentes, **caractérisé en ce que** la forme du patch est adaptée à la forme du nez, des lèvres, de la zone comprise entre le nez et la lèvre supérieure, ou pour traiter les pattes d'oie du coin externe de l'oeil, les poches en-dessous de l'oeil, ou les rides du front.

38. Patch pour une utilisation selon un procédé de traitement selon l'une quelconque des revendications précédentes, constitué d'une matrice polymérique déposée sur un support, ladite matrice étant auto-adhésive sur peau sèche, et contenant au moins un composé cosmétiquement actif, et au moins un composé hydroabsorbant, ledit patch étant apte à être appliqué sélectivement :
a) en mode nettoyage sur peau sèche, le décollement du patch après application permettant, sous l'action mécanique résultant de l'adhérence du patch, de débarrasser la peau des impuretés qu'elle contient, ou
b) en mode traitement à l'état mouillé, le (ou les) composé(s) hydroabsorbants permettant d'une part, en asséchant partiellement la peau, de conserver une adhérence de la matrice sur la peau, laquelle adhérence est réduite par rapport à l'adhérence de la matrice sur peau sèche, et d'autre part, en absorbant de l'eau, de resolubiliser tout ou partie des composés cosmétiquement actifs contenus dans la matrice pour les mettre en contact avec la peau pendant toute la durée d'application, en formant une couche interface entre la matrice et la peau.

39. Patch selon la revendication 38 **caractérisé en ce que** la matrice contient des particules d'hydroabsorbants, et éventuellement de composés actifs, ou des agglomérats de telles particules, émergeant à l'état sec par rapport à la surface moyenne de la matrice, et aptes à former ladite couche interface à l'état mouillé.

40. Patch selon la revendication 39 **caractérisé en ce que** lesdites particules ou agglomérats de particules font saillie par rapport à la surface moyenne de la matrice, d'une hauteur allant de 10 microns à 100 microns, et de préférence, de 10 µm à 70 µm.

41. Patch selon l'une quelconque des revendications 38 à 40 **caractérisé en ce que** la couche interface formée à l'état mouillé à une épaisseur comprise entre 50µm et 100 µm.

## Patentansprüche

1. Verfahren zur kosmetischen Behandlung der Haut mit einem Pflaster, das aus einer auf einem Träger abgeschiedenen polymeren Matrix besteht, wobei die Matrix auf der trockenen Haut selbsthaftend ist und mindestens eine kosmetisch aktive Verbindung und mindestens eine wasserabsorbierende Verbindung enthält, wobei das Verfahren darin besteht, das Pflaster selektiv anzuwenden:
a) im Reinigungsmodus auf die trockene Haut, wobei die Haut beim Ablösen des Pflasters nach der Anwendung auf Grund der Haftung des Pflasters unter der mechanischen Einwirkung von Unreinheiten, die sie aufweist, befreit werden kann, oder
b) im Behandlungsmodus im feuchten Zustand, wobei die wasserabsorbierende Verbindung (oder die wasserabsorbierenden Verbindungen) einerseits durch teilweises Austrocknen der Haut die Haftung der Matrix auf der Haut aufrecht erhalten können, wobei die Haftung im Vergleich zu der Haftung der Matrix auf der trockenen Haut vermindert ist, und andererseits durch die Absorption von Wasser die in der Matrix enthaltenen kosmetisch aktiven Verbindungen zum Teil oder vollständig solubilisieren können, damit sie während der gesamten Dauer der Anwendung mit der Haut in Kontakt sind.

2. Verfahren nach Anspruch 1, **dadurch gekennzeichnet, dass** es sich bei dem Träger um einen okklusiven Träger handelt.

3. Verfahren nach Anspruch 1 oder 2, **dadurch gekennzeichnet, dass** der Träger aus einem thermoplastischen Stoff, der unter Polyethylenen mit hoher oder niedriger Dichte, Polypropylenen, Polyvinylchloriden, Copolymeren von Ethylen und Vinylacetat, Polyurethanen und Polyestern ausgewählt ist, aus einem Komplex dieser Stoffe oder aus mit einer Aluminiumfolie beschichtetem Polyethylenterephthalat hergestellt ist.

4. Verfahren nach Anspruch 1, **dadurch gekennzeichnet, dass** es sich bei dem Träger um einen nicht okklusiven Träger handelt.

5. Verfahren nach Anspruch 4, **dadurch gekennzeichnet, dass** der Träger aus Papier, einem porösen oder perforierten thermoplastischen Stoff, einem gewebten Stoff, einem Vliesstoff oder einem perforierten Vliesstoff hergestellt ist.

6. Verfahren zur Behandlung nach einem der vorhergehenden Ansprüche, **dadurch gekennzeichnet, dass** es in einer Anwendung im Reinigungsmodus und einer anschließenden Anwendung im Behandlungsmodus besteht.

7. Verfahren nach einem der Ansprüche 1 bis 5, **dadurch gekennzeichnet, dass** es in einer Anwendung im Behandlungsmodus und einer anschließenden Anwendung im Reinigungsmodus besteht.

8. Verfahren zur Behandlung nach einem der vorhergehenden Ansprüche, **dadurch gekennzeichnet, dass** die kosmetisch aktive Verbindung unter Emollientien, Hydratisierungsmitteln, reizlindemden Wirkstoffen, Keratolytika, abschilfernden Wirkstoffen, wundheilenden Wirkstoffen, regenerierenden Wirkstoffen, Wirkstoffen gegen Falten, straffenden Wirkstoffen, Wirkstoffen zum Sonnenschutz, beruhigenden Wirkstoffen, Selbstbräunern, aufhellenden Wirkstoffen oder deren Gemischen ausgewählt ist.

9. Verfahren zur Behandlung nach einem der vorhergehenden Ansprüche, **dadurch gekennzeichnet, dass** die kosmetisch aktive Verbindung in der polymeren Matrix in Partikelform dispergiert ist.

10. Verfahren zur Behandlung nach einem der vorhergehenden Ansprüche, **dadurch gekennzeichnet, dass** die Anwendung im Reinigungsmodus im Wechsel mit der Anwendung im Behandlungsmodus erfolgt.

11. Verfahren zur Behandlung nach dem vorhergehenden Anspruch, **dadurch gekennzeichnet, dass** mehrere Anwendungen im Reinigungsmodus im Wechsel mit mehreren Anwendungen im Behandlungsmodus durchgeführt werden.

12. Verfahren zur Behandlung nach einem der vorhergehenden Ansprüche, **dadurch gekennzeichnet, dass** die Anwendung im Reinigungsmodus während eine Zeitspanne von etwa 5 Sekunden bis etwa 5 Minuten erfolgt.

13. Verfahren zur Behandlung nach einem der vorhergehenden Ansprüche, **dadurch gekennzeichnet, dass** die Anwendung im Behandlungsmodus während eine Zeitspanne von etwa 5 Minuten bis etwa 30 Minuten erfolgt.

14. Verfahren zur Behandlung nach einem der vorhergehenden Ansprüche, **dadurch gekennzeichnet, dass** das Pflaster auf der trockenen Haut ein Haftvermögen im Bereich von etwa 300 bis etwa 800 g/cm² aufweist.

15. Verfahren zur Behandlung nach einem der vorhergehenden Ansprüche, **dadurch gekennzeichnet, dass** das Pflaster im feuchten Zustand ein Haftvermögen im Bereich von etwa 20 bis etwa 300 g/cm² aufweist.

16. Verfahren zur Behandlung nach einem der vorhergehenden Ansprüche, **dadurch gekennzeichnet, dass** die kosmetisch aktive Verbindung unter wasserlöslichen Wirkstoffen, wie Ascorbinsäure und ihren biologisch kompatiblen Salzen, Enzymen, Antibiotika, Komponenten mit straffender Wirkung, α-Hydroxysäuren und ihren Salzen, Polyhydroxycarbonsäuren, Saccharosen und ihren Derivaten, Harnstoff, Aminosäuren, Oligopeptiden, wasserlöslichen Pflanzenextrakten und Hefeextrakten, Proteinhydrolysaten, Hyaluronsäure, Mucopolysacchariden, den Vitaminen B₂, B₆, H und PP, Panthenol, Folsäure, Acetylsalicylsäure, Allantoin, Glycyrrhetinsäure, Kojisäure und Hydrochinon, ausgewählt ist.

17. Verfahren zur Behandlung nach einem der vorhergehenden Ansprüche, **dadurch gekennzeichnet, dass** die kosmetisch aktive Verbindung unter fettlöslichen Verbindungen, wie D-α-Tocopherol, DL-α-Tocopherol, D-α-Tocopherylacetat, DL-α-Tocopherylacetat, Ascorbylpalmitat, Vitamin F und Glyceriden von Vitamin F, D-Vitaminen, Vitamin D₂, Vitamin D₃, Retinol, Retinolestern, Retinolpalmitat, Retinolpropionat, β-Carotin, D-Panthenol, Farnesol, Farnesylacetat, Jojobaöl und Öl von schwarzen Johannisbeeren, die reich an essentiellen Fettsäuren sind, Keratolytika, wie Salicylsäure, ihren Salzen und ihren Estem, 5-(n-Octanoyl)-salicylsäure und ihren Estern, Alkylestern von α-Hydroxysäuren, wie Citronensäure, Milchsäure oder Glykolsäure, Asiatsäure, Madecassischer Säure, Asiaticosid, dem Totalextrakt von Centella asiatica, β-Glycyrrhetinsäure, α-Bisabolol, Ceramiden, wie 2-Oleoylamino-1,3-octadecan, Phytantriol, Sphingomyelin von Milch, Phospholipiden marinen Ursprungs, die reich an mehrfach ungesättigten essentiellen Fettsäuren sind, Ethoxychin, Rosmarinextrakt, Melisseextrakt, Quercetin, dem Extrakt von getrockneten Mikroalgen und steroidalen entzündungshemmenden Wirkstoffen, ausgewählt ist.

18. Verfahren zur Behandlung nach Anspruch 8, **dadurch gekennzeichnet, dass** das Keratolytikum unter α- und β-Hydroxycarbonsäuren oder β-Ketocarbonsäuren, ihren Salzen, Amiden oder Estern und insbesondere α-Hydroxysäuren, wie Glykolsäure, Milchsäure, Weinsäure, Äpfelsäure, Citronensäure, Mandelsäure und allgemein Fruchtsäuren, sowie β-Hydroxysäuren, wie Salicylsäure und ihren Derivaten und insbesondere ihren alkylierten Derivaten, wie 5-(n-Octanoyl)-salicylsäure ausgewählt ist.

19. Verfahren zur Behandlung nach Anspruch 8, **dadurch gekennzeichnet, dass** das Emolliens unter flüchtigen oder nicht flüchtigen Siliconen, Polydiorganosiloxan-Polyoxyalkylen-Copolymeren, natürlichen oder synthetischen Ölen, wie Mineralölen, pflanzlichen und tierischen Ölen, Fetten und Wachsen, Fettalkoholen, Fettsäuren und ihren Estern, Estern und Ethern von (Poly)alkylenglykolen, Kohlenwasserstoffen, wie Isohexadecan, Petrolatum und Squalan, Lanolinalkohol und seinen Derivaten, tierischen oder pflanzlichen Triglyceriden und Stearylalkohol ausgewählt ist.

20. Verfahren zur Behandlung nach Anspruch 8, **dadurch gekennzeichnet, dass** das Hydratisierungsmittel unter Glycerin, Sorbit und seinen Derivaten, Propylenglykol, Dipropylenglykol, Butylenglykol, D-Panthenol oder DL-Panthenol und ihren Derivaten ausgewählt ist.

21. Verfahren zur Behandlung nach Anspruch 8, **dadurch gekennzeichnet, dass** der reizlindernde Wirkstoff unter Allantoin ausgewählt ist.

22. Verfahren zur Behandlung nach Anspruch 8, **dadurch gekennzeichnet, dass** der abschilfernde Wirkstoff unter alkylierten Derivaten von Salicylsäure, 5-(n-Octanoyl)-salicylsäure, α- und β-Hydroxycarbonsäuren oder β-Ketocarbonsäuren und ihren Salzen, Amiden oder Estern ausgewählt ist.

23. Verfahren zur Behandlung nach Anspruch 8, **dadurch gekennzeichnet, dass** die Wirkstoffe gegen Falten und die straffenden Wirkstoffe unter α- und β-Hydroxycarbonsäuren oder β-Ketocarbonsäuren und ihren Salzen, Amiden oder Estern, Salicylsäure, Ascorbinsäure, Azelainsäure, Retinol und den Derivaten dieser Verbindungen ausgewählt sind.

24. Verfahren zur Behandlung nach Anspruch 8, **dadurch gekennzeichnet, dass** der Wirkstoff zum Sonnenschutz unter UV-A-Filtern und/ oder UV-B-Filtern ausgewählt ist.

25. Verfahren zur Behandlung nach Anspruch 8, **dadurch gekennzeichnet, dass** der beruhigende Wirkstoff unter α-Bisabolol ausgewählt ist.

26. Verfahren zur Behandlung nach Anspruch 8, **dadurch gekennzeichnet, dass** der selbstbräunende Wirkstoff unter Dihydroxyaceton und den Derivaten von Hydroxynaphthochinon ausgewählt ist.

27. Verfahren zur Behandlung nach Anspruch 8, **dadurch gekennzeichnet, dass** der aufhellende Wirkstoff unter Citronensäure, Hydrochinon, Kojisäure und Ascorbinsäure ausgewählt ist.

28. Verfahren zur Behandlung nach einem der vorhergehenden Ansprüche, **dadurch gekennzeichnet, dass** die polymere Matrix des Pflasters ein Acrylpolymer oder Vinylpolymer enthält.

29. Verfahren zur Behandlung nach einem der vorhergehenden Ansprüche, **dadurch gekennzeichnet, dass** die wasserabsorbierende Verbindung unter superabsorbierenden vernetzten Polyacrylaten, Polyvinylalkohol, Carboxyvinylpolymeren, halbsynthetischen Derivaten von Cellulose, Stärken, Biogummen, wie Xanthangummi, Guargummi, Gummi arabicum und Traganth, Biosacchariden, Skleroglucanen, Casein, Phycokolloiden, wie Alginaten, Gelatine und Baumwollfasern ausgewählt ist.

30. Verfahren zur Behandlung nach einem der vorhergehenden Ansprüche, **dadurch gekennzeichnet, dass** das Pflaster mindestens einen Fortsatz aufweist, der geeignet ist, einen Bereich zu bilden, an dem das Pflaster zum Ablösen gegriffen werden kann.

31. Verfahren zur Behandlung nach einem der vorhergehenden Ansprüche, **dadurch gekennzeichnet, dass** die polymere Matrix gefärbt ist, um im Reinigungsmodus die mit der haftenden Oberfläche entfernten Unreinheiten mengenmäßig und/oder qualitativ durch persönlichen Augenschein erfassen zu können.

32. Verfahren zur Behandlung nach Anspruch 31, **dadurch gekennzeichnet, dass** die polymere Matrix des Pflasters durch Einarbeitung farbiger Pigmente gefärbt ist.

33. Verfahren zur Behandlung nach Anspruch 31 oder 32, **dadurch gekennzeichnet, dass** die polymere Matrix dunkel gefärbt ist, damit ein ausreichender Kontrast erzielt wird, um die von der Haut entfernten Unreinheiten sichtbar zu machen.

34. Verfahren zur Behandlung nach einem der vorhergehenden Ansprüche, **dadurch gekennzeichnet, dass** die polymere Matrix eine Lage aufweist, die aus einem porösen oder perforierten thermoplastischen Stoff, einem gewebten Stoff, einem Vliesstoff oder einem perforierten Vliesstoff hergestellt ist.

35. Verfahren zur Behandlung nach einem der vorhergehenden Ansprüche, **dadurch gekennzeichnet, dass** das Pflaster eine Schutzfolie aufweist, die vor der Anwendung des Pflasters abgezogen werden kann und die für die Verbindung(en), die in der Zusammensetzung der polymeren Schicht vorliegt (vorliegen), undurchlässig ist.

36. Verfahren zur Behandlung nach Anspruch 35, **dadurch gekennzeichnet, dass** die Schutzfolie des Pflasters aus zwei Teilen besteht, die sich in einem Abschnitt in der Mitte des Pflasters überlappen.

37. Verfahren zur Behandlung nach einem der vorhergehenden Ansprüche, **dadurch gekennzeichnet, dass** die Form des Pflasters an die Form der Nase, der Lippen oder des Bereichs zwischen der Nase und der Oberlippe oder zur Behandlung von Krähenfüßen am äußeren Augenwinkel, Tränensäcken unter dem Auge oder Stirnfalten angepasst ist.

38. Pflaster für eine Verwendung nach einem Verfahren zur Behandlung nach einem der vorhergehenden Ansprüche, das aus einer auf einem Träger abgeschiedenen polymeren Matrix besteht, wobei die Matrix auf der trockenen Haut selbsthaftend ist und mindestens eine kosmetisch aktive Verbindung und mindestens eine wasserabsorbierende Verbindung enthält, wobei das Pflaster befähigt ist, selektiv angewendet zu werden:
a) im Reinigungsmodus auf die trockene Haut, wobei die Haut beim Ablösen des Pflasters nach der Anwendung auf Grund der Haftung des Pflasters unter der mechanischen Einwirkung von Unreinheiten, die sie aufweist, befreit werden kann, oder
b) im Behandlungsmodus im feuchten Zustand, wobei die wasserabsorbierende Verbindung (oder die wasserabsorbierenden Verbindungen) einerseits durch teilweises Austrocknen der Haut die Haftung der Matrix auf der Haut aufrecht erhalten können, wobei die Haftung im Vergleich zu der Haftung der Matrix auf der trockenen Haut vermindert ist, und andererseits durch die Absorption von Wasser die in der Matrix enthaltenen kosmetisch aktiven Verbindungen zum Teil oder vollständig solubilisieren können, damit sie während der gesamten Dauer der Anwendung mit der Haut in Kontakt sind, wobei eine Grenzschicht zwischen der Matrix und der Haut gebildet wird.

39. Pflaster nach Anspruch 38, **dadurch gekennzeichnet, dass** die Matrix wasserabsorbierende Partikel und gegebenenfalls Partikel von Wirkstoffverbindungen oder Agglomerate derartiger Partikel enthält, die im trockenen Zustand, bezogen auf die mittlere Oberfläche der Matrix, vorragen und im feuchten Zustand zur Bildung der Grenzschicht befähigt sind.

40. Pflaster nach Anspruch 39, **dadurch gekennzeichnet, dass** die Partikel oder die Partikelagglomerate, bezogen auf die mittlere Oberfläche der Matrix, mit einer Höhe vorragen, die im Bereich von 10 Mikrometer bis 100 Mikrometer und vorzugsweise von 10 µm bis 70 µm liegt.

41. Pflaster nach einem der Ansprüche 38 bis 40, **dadurch gekennzeichnet, dass** die im feuchten Zustand gebildete Grenzschicht eine Dicke im Bereich von 50 µm bis 100 µm aufweist.

## Claims

1. Cosmetic skin treatment method using a patch consisting of a polymeric matrix deposited on a support, the said matrix being self-adhesive with respect to dry skin and containing at least one cosmetically active compound and at least one water-absorbent compound, the said method consisting in selectively applying the patch:
a) in cleansing mode to dry skin, the process of pulling off the patch after application making it possible, due to the mechanical action resulting from the adhesion of the patch, to rid the skin of the impurities that it contains; or
b) in treatment mode in the wet state, the water-absorbent compound(s) making it possible, on the one hand, by partially drying the skin, to maintain some adhesion of the matrix to the skin, this adhesion being less than the adhesion of the matrix to dry skin, and, on the other hand, by absorbing water, to redissolve all or some of the cosmetically active compounds contained in the matrix, in order to bring them into contact with the skin throughout the duration of the application.

2. Method according to Claim 1, **characterized in that** the support is occlusive.

3. Method according to Claim 1 or 2, **characterized in that** the support is made of a thermoplastic chosen from high-density and low-density polyethylenes, polypropylenes, polyvinyl chlorides, ethylene and vinyl acetate copolymers, polyurethanes and polyesters, or made of a complex of such materials, or polyethylene terephthalate covered with an aluminium foil.

4. Method according to Claim 1, **characterized in that** the support is non-occlusive.

5. Method according to Claim 4, **characterized in that** the support is made of paper, a porous or perforated thermoplastic, a woven fabric, a nonwoven fabric or a perforated nonwoven fabric.

6. Treatment method according to any one of the preceding claims, **characterized in that** it consists of an application in cleansing mode followed by an application in treatment mode.

7. Method according to any one of Claims 1 to 5, **characterized in that** it consists of an application in treatment mode followed by an application in cleansing mode.

8. Treatment method according to any one of the preceding claims, **characterized in that** the cosmetically active compound is chosen from emollients, moisturizers, softening agents, keratolytic agents, desquamating agents, cicatrizing agents, regenerating agents, anti-wrinkle agents, tautening agents, sunscreen agents, soothing agents, self-tanning agents, lightening agents or mixtures thereof.

9. Treatment method according to any one of the preceding claims, **characterized in that** the cosmetically active compound is dispersed in the polymeric matrix in particulate form.

10. Treatment method according to any one of the preceding claims, **characterized in that** the application in cleansing mode is carried out alternately with the application in treatment mode.

11. Treatment method according to the preceding claim, **characterized in that** a number of applications in cleansing mode are carried out alternately with a number of applications in treatment mode.

12. Treatment method according to any one of the preceding claims, **characterized in that** the application in cleansing mode is carried out for a period ranging from about 5 seconds to about 5 minutes.

13. Treatment method according to any one of the preceding claims, **characterized in that** the application in treatment mode is carried out for a period ranging from about 5 minutes to about 30 minutes.

14. Treatment method according to any one of the preceding claims, **characterized in that** the patch has an adhesiveness with respect to dry skin of between about 300 and about 800 g/cm².

15. Treatment method according to any one of the preceding claims, **characterized in that** the patch has an adhesiveness in the wet state of between about 20 and about 300 g/cm².

16. Treatment method according to any one of the preceding claims, **characterized in that** the cosmetically active compound is chosen from water-soluble active agents such as ascorbic acid and its biologically compatible salts, enzymes, antibiotics, components having a tautening effect, α-hydroxy acids and their salts, hydroxylated polyacids, sucroses and their derivatives, urea, amino acids, oligopeptides, water-soluble plant and yeast extracts, protein hydrolysates, hyaluronic acid, mucopolysaccharides, vitamins B₂, B₆, H and PP, panthenol, folic acid, acetylsalicylic acid, allantoin, glycyrrhetic acid, kojic acid and hydroquinone.

17. Treatment method according to any one of the preceding claims, **characterized in that** the cosmetically active compound is chosen from liposoluble compounds such as D-α-tocopherol, DL-α-tocopherol, D-α-tocopherol acetate, DL-α-tocopherol acetate, ascorbyl palmitate, vitamin F and vitamin F glycerides, vitamins D, vitamin D₂, vitamin D₃, retinol, retinol esters, retinol palmitate, retinol propionate, β-carotene, D-panthenol, famesol, farnesyl acetate; jojoba and blackcurrant oils rich in essential fatty acids; keratolytic agents such as salicylic acid, its salts and its esters, 5-(n-octanoyl)salicylic acid and its esters, alkyl esters of α-hydroxy acids such as citric acid, lactic acid, glycolic acid; asiatic acid, madecassic acid, asiaticoside, total extract of centella asiatica, β-glycyrrhetinic acid, α-bisabolol, ceramides such as 2-oleoylamino-1,3-octadecane; phytanetriol, milk sphingomyelin, phospholipids of marine origin, rich in polyunsaturated essential fatty acids, ethoxyquin; extract of rosemary, extract of melissa, quercetin, extract of dried microalgae, and steroidal anti-inflammatory drugs.

18. Treatment method according to Claim 8, **characterized in that** the keratolytic agent is chosen from: alpha- and beta-hydroxycarboxylic acids or beta-ketocarboxylic acids, their salts, amides or esters, and more particularly alpha-hydroxy acids such as glycolic acid, lactic acid, tartaric acid, malic acid, citric acid, mandelic acid and, in general, fruit acids and beta-hydroxy acids such as salicylic acid and its derivatives, especially alkylated derivatives such as 5-(n-octanoyl)salicylic acid.

19. Treatment method according to Claim 8, **characterized in that** the emollient is chosen from: volatile and non-volatile silicones, polydiorgano-siloxane-polyoxyalkylene copolymers; natural or synthetic oils such as mineral, plant and animal oils; fats and waxes; fatty alcohols and acids, and their esters; esters and ethers of (poly)alkylene glycols; hydrocarbons such as isohexadecane, petrolatum and squalane; lanolin alcohol and its derivatives; animal and plant triglycerides; stearyl alcohol.

20. Treatment method according to Claim 8, **characterized in that** the moisturizer is chosen from: glycerol, sorbitol and its derivatives, propylene glycol, dipropylene glycol, butylene glycol, D- or DL-panthenol and their derivatives.

21. Treatment method according to Claim 8, **characterized in that** the softening agent is chosen from: allantoin.

22. Treatment method according to Claim 8, **characterized in that** the desquamating agent is chosen from: alkylated derivatives of salicylic acid, 5-(n-octanoyl)salicylic acid, alpha- and beta-hydroxycarboxylic acids or beta-ketocarboxylic acids, their salts, amides or esters.

23. Treatment method according to Claim 8, **characterized in that** the anti-wrinkle and tautening agents are chosen from: alpha- and beta-hydroxycarboxylic acids or beta-ketocarboxylic acids, their salts, amides or esters; salicylic acid, ascorbic acid, azelaic acid, retinol and their derivatives.

24. Treatment method according to Claim 8, **characterized in that** the sunscreen agent is chosen from UV-A filters and/or UV-B filters.

25. Treatment method according to Claim 8, **characterized in that** the soothing agent is chosen from: α-bisabolol.

26. Treatment method according to Claim 8, **characterized in that** the self-tanning agent is chosen from: dihydroxyacetone and derivatives of hydroxynaphthoquinone.

27. Treatment method according to Claim 8, **characterized in that** the lightening agent is chosen from: citric acid, hydroquinone, kojic acid and ascorbic acid.

28. Treatment method according to any one of the preceding claims, **characterized in that** the polymeric matrix of the patch contains an acrylic or vinyl polymer.

29. Treatment method according to any one of the preceding claims, **characterized in that** the water-absorbent compound is chosen from superabsorbent crosslinked polyacrylates; polyvinyl alcohol; carboxyvinyl polymers; semi-synthetic derivatives of cellulose; starches; biogums such as xanthan gum, guar gum, gum arabic and gum tragacanth, biosaccharides, scleroglucans; casein; phycocolloids, such as alginates; gelatin and cotton fibres.

30. Treatment method according to any one of the preceding claims, **characterized in that** the patch comprises at least one excrescence, capable of forming a gripping region in order to pull it off.

31. Treatment method according to any one of the preceding claims, **characterized in that** the polymeric matrix is coloured so as to be able, *de visu,* to quantify and/or qualify the impurities removed by the said adhesive surface in cleansing mode.

32. Treatment method according to Claim 31, **characterized in that** the polymeric matrix of the patch is coloured by the incorporation of colour pigments.

33. Treatment method according to Claim 31 or 32, **characterized in that** the colour of the polymeric matrix is dark so as to produce sufficient contrast to show up the impurities extracted from the skin.

34. Treatment method according to any one of the preceding claims, **characterized in that** the polymeric matrix comprises a mesh, made of a porous or perforated thermoplastic or a woven fabric or a nonwoven fabric or a perforated nonwoven fabric.

35. Treatment method according to any one of the preceding claims, **characterized in that** the patch comprises a protective film which can be peeled off before the patch is applied and is impermeable to the compound(s) contained in the composition of the polymeric layer.

36. Treatment method according to Claim 35, **characterized in that** the protective film of the patch consists of two parts superposed on a central portion of the patch.

37. Treatment method according to any one of the preceding claims, **characterized in that** the shape of the patch is tailored to the shape of the nose, the lips, the region between the nose and the upper lip, or for treating crow's feet in the outer corners of the eyes, bags under the eyes or forehead wrinkles.

38. Patch for use in accordance with a treatment method according to any one of the preceding claims, consisting of a polymeric matrix deposited on a support, the said matrix being self-adhesive with respect to dry skin and containing at least one cosmetically active compound and at least one water-absorbent compound, the said patch being capable of being applied selectively:
a) in cleansing mode to dry skin, the process of pulling off the patch after application making it possible, due to the mechanical action resulting from the adhesion of the patch, to rid the skin of the impurities that it contains; or
b) in treatment mode in the wet state, the water-absorbent compound(s) making it possible, on the one hand, by partially drying the skin, to maintain some adhesion of the matrix to the skin, this adhesion being less than the adhesion of the matrix to dry skin, and, on the other hand, by absorbing water, to redissolve all or some of the cosmetically active compounds contained in the matrix, in order to bring them into contact with the skin throughout the duration of the application, forming an interface layer between the matrix and the skin.

39. Patch according to Claim 38, **characterized in that** the matrix contains particles of water absorbents, and optionally of active compounds, or agglomerates of such particles, emerging in the dry state from the mean surface of the matrix and capable of forming the said interface layer in the wet state.

40. Patch according to Claim 39, **characterized in that** the said particles or agglomerates of particles project from the mean surface of the matrix by a height ranging from 10 microns to 100 microns, and preferably by 10 µm to 70 µm.

41. Patch according to any one of Claims 38 to 40, **characterized in that** the interface layer formed in the wet state has a thickness of between 50 µm and 100 µm.
